Europäisches Patentamt

European Patent Office

Office européen des brevets

(11) Veröffentlichungsnummer: **0 277 304**
**A1**

(12) # EUROPÄISCHE PATENTANMELDUNG

(21) Anmeldenummer: **87117596.4**

(22) Anmeldetag: **27.11.87**

(51) Int. Cl.⁴ **G01R 33/00 , A61B 5/04**

(30) Priorität: **09.12.86 DE 3641945**

(43) Veröffentlichungstag der Anmeldung:
**10.08.88 Patentblatt 88/32**

(84) Benannte Vertragsstaaten:
**DE FR IT NL**

(71) Anmelder: **Siemens Aktiengesellschaft Berlin
und München**
**Wittelsbacherplatz 2**
**D-8000 München 2(DE)**

(72) Erfinder: **Naser, Georg, Dipl.-Ing. (FH)**
**Karlstrasse 10**
**D-8502 Zirndorf(DE)**
Erfinder: **Reichelsdörfer, Peter**
**Bischoff-Fischer-Strasse 44**
**D-7080 Aalen(DE)**
Erfinder: **Schneider, Siegfried, Dr.**
**Kulmbacher Strasse 33**
**D-8520 Erlangen(DE)**

(54) **Haltevorrichtung für einen Messkryostaten.**

(57) Die Haltevorrichtung (1) umfaßt zwei vertikale Stativsäulen (3), die an ihren oberen Enden über eine Querstrebe (5) verbunden sind. An jeder Stativsäule (3) ist ein Laufkasten (17) verschiebbar angeordnet. Die beiden Laufkästen (17) sind über einen Bügel (19) miteinander verbunden. An dem Bügel (19) ist ein Kreisscheibensegment (21) angebracht, das mindestens eine Nut (31) aufweist. Entlang der Nut (31) kann der Meßkryostat (15) um einen Winkel Alpha geschwenkt werden. Der Bügel (19) ist mit Hilfe von Kreisscheibensegmenten (21) und Bolzen (23) drehbar ausgelegt. Der Meßkryostat (15) ist mit Hilfe eines Schlittens (33) in Radialrichtung des Kreisringsegments (29) verschiebbar. Außerdem ist der Meßkryostat (15) um seine Zentralachse (35) drehbar.

Vorteil der Haltevorrichtung (1) ist die leichte Höhenverstellbarkeit des Meßkryostaten (15) zur Anpassung an sitzende oder liegende Patienten und die Möglichkeit zum Abfahren der Applikationsfläche (37) entlang einer Kugeloberfläche, deren Mittelpunkt (P) im Kopf oder Thorax des Patienten liegt.

FIG 1

### Haltevorrichtung für einen Meßkryostaten

Die Erfindung betrifft eine Haltevorrichtung für einen Meßkryostaten zur Erfassung biomagnetischer Signale.

An die Stabilität solcher Haltevorrichtungen werden hohe Ansprüche gestellt, da der Meßkryostat eine magnetische Meßspule umfaßt, bei welcher Bewegungen bis hinunter in den Mikrometerbereich unerwünscht sind. Gleichzeitig soll eine möglichst hohe Flexibilität bezüglich der Handhabbarkeit und Einstellbarkeit des Meßkryostaten mit Meßspule gegeben sein.

Zu dieser Forderung gehört eine leichte Höhenverstellung für unterschiedliche Meßpositionen bei liegendem oder sitzendem Patienten oder für Arbeiten am Kryostaten, wie z.B. beim Nachfüllen von flüssigem Helium. Weiterhin gehört dazu eine einfache Bewegbarkeit des Meßkryostaten auf einer Kugeloberfläche, deren Mittelpunkt innerhalb des Kopfes oder im Thorax des Patienten liegt. Dabei soll der Radius für die abzufahrende Kugeloberfläche nach Möglichkeit verstellbar sein. Ferner soll die Meßspule relativ zum interessierenden Meßgebiet am Kopf oder Thorax des Patienten durch Drehung der Kryostatenachse zu verstellen sein. Auch sollte noch die Bedingung erfüllt sein, daß die Haltvorrichtung möglichst frei von mechanischen Resonanzen ist. Schließlich sollte sie auch einen guten Zugang zum Patienten und zum Kryostaten zwecks Wartungsarbeiten ermöglichen. Daneben muß der in der Medizintechnik übliche Sicherheitsstandard zum Schutz des Patienten erreicht werden.

Ein Meßkryostat der hier betrachteten Art für die Messung biomagnetischer Signale ist beispielsweise aus dem Aufsatz von D. Crum "The Design and Use of Dewars for Biomagnetic Measurements" im Buch "Biomagnetism, Applications and Theory", herausgegeben von H. Weinberg, G. Stroink, T. Katila-Pergamon Press, New York 1985, S. 21-34, bekannt.

Aufgabe der Erfindung ist es, eine Haltevorrichtung der eingangs genannten Art anzugeben, bei der die genannten Forderungen möglichst gut erfüllt sind.

Diese Aufgabe wird erfindungsgemäß dadurch gelöst, daß die Haltevorrichtung zwei Stativsäulen umfaßt, an welchen jeweils ein Laufkasten verschiebbar angeordnet ist, und daß die Laufkästen über einen drehbar gelagerten Bügel miteinander verbunden sind, an welchem der Meßkryostat in der Bügelebene schwenkbar befestigt ist.

Durch die verschiebbar angeordneten Laufkästen wird dabei eine einfache Höhenverstellung des Kryostaten erreicht. Der drehbar gelagerte Bügel in Zusammenhang mit der Schwenkbarkeit in Bügelebene ermöglicht das Abfahren des Meßkryostaten auf einer Kugeloberfläche. Deren Mittelpunkt kann so gewählt werden, daß er im Inneren des Kopfes oder des Thorax des zu untersuchenden Patienten liegt. Bei Befestigung der beiden Stativsäulen direkt auf dem bauseitigen Fundament vergleichsweise großer Masse wird eine hohe Stabilität gegenüber Schwingungen erreicht.

Weitere Vorteile und Ausgestaltungen der Erfindung ergeben sich aus der Beschreibung eines Ausführungsbeispiels anhand der Figuren. Es zeigen:

Fig. 1 eine Seitenansicht einer Haltevorrichtung für einen Meßkryostaten und

Fig. 2 eine Aufsicht auf die Haltevorrichtung gemäß Fig. 1.

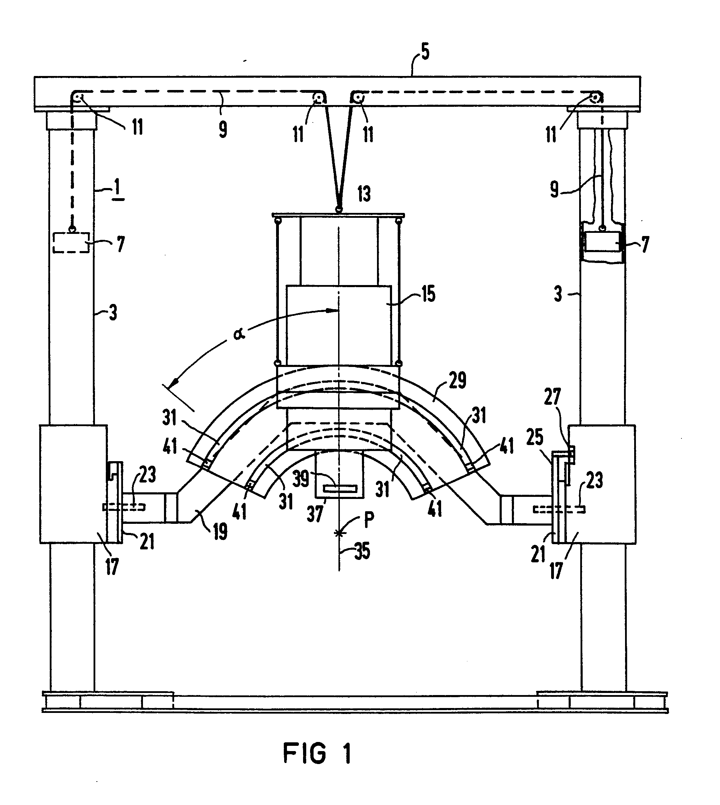

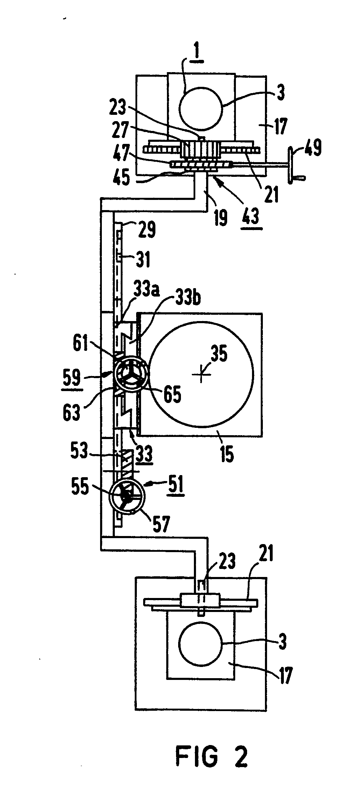

In Fig. 1 und 2 ist eine Haltevorrichtung 1 gezeigt, die zwei vertikale Stativsäulen 3 besitzt, die an ihren oberen Enden über eine Querstrebe 5 miteinander verbunden sind. Die Stativsäulen 3 sind innen hohl und dadurch geeignet, jeweils ein innen verschiebbares Gegengewicht 7 aufzunehmen. Jedes Gegengewicht 7 ist über ein Seil 9, welches über Umlenkrollen 11 bis zur Mitte der hohlen Querstrebe 5 läuft, mit einer gemeinsamen Öse 13 verbunden, welche auf der Halterung für den Meßkryostaten befestigt ist . Die beiden Seilenden können auch einzeln an der Kryostatenhalterung befestigt sein. Dieser Meßkryostat 15 ist an sich bekannt. Er enthält eine durch flüssiges Helium gekühlte Meßspule und dient zur Erfassung biomagnetischer Signale eines Patienten, z.B. verursacht von Gehirnströmen.

An den Stativsäulen 3 ist jeweils ein Laufkasten 17 höhenverschiebbar angebracht. Die Laufkästen 17 sind über einen Bügel 19 miteinander verbunden. Der Bügel 19 weist an seinen Enden je ein Kreisscheibensegment 21 auf, das drehbar um einen an dem zugeordneten Laufkasten 17 befestigten Bolzen 23 gelagert ist. Am Umfang mindestens eines der Kreisscheibensegmente 21 ist ein Zahnkranz 25 vorgesehen, an welchem ein Zahnradantrieb 27 angreift. Der Bügel 19 kann somit um die durch die Bolzen 23 festgelegte Achse geschwenkt werden.

In der Mitte des Bügels 19 ist ein Kreisringsegment 29 angebracht. Das Kreisringsegment 29 ist mit einer oder bevorzugt mehreren parallelen kreissegmentförmigen Nuten 31 versehen. An den Nuten 31 ist der Meßkryostat 15 über an ihm befestigte Nutensteine schwenkbar gelagert. Der Schwenkwinkel bezüglich der Vertikalen ist mit Alpha bezeichnet. Die Verstellung um den Winkel Alpha erfolgt über ein Stirnradgetriebe. Das Kreisringsegment 29 ist beim Schwenken jeweils parallel zur Bügelebene ausgerichtet.

Über einen Schlitten 33 ist der Meßkryostat 15 in seiner Höhe relativ zum Kreisringsegment 29 verschiebbar. Die Verschiebung erfolgt dabei immer in radialer Richtung, also in Richtung des Radius des Kreissegments 29. Über ein Gleitlager (nicht gezeigt) ist der Meßkryostat 15 weiterhin um seine Zentralachse 35 drehbar.

Mit Hilfe der zuvor beschriebenen Gegengewichte 7 in Verbindung mit den Seilen 9 und den Umlenkrollen 11 ist die Gesamtheit von Meßkryostat 15, Kreisringsegment 29, Bügel 19 und Laufkästen 17 in der Höhe, also relativ zu den Stativsäulen 3, verschiebbar. Die Querstrebe 5 dient also neben der mechanischen Versteifung gleichzeitig als Brücke für einen Gewichtsausgleich zwischen dem Meßkryostatengewicht einerseits und den in den Stativsäulen 3 gleitenden Gegengewichten 7 andererseits. Diese Höhenverstellung dient dazu, den Meßkryostaten 15 mit seiner Applikationsfläche 37, hinter der unmittelbar die Meßspule 39 liegt, dicht sowohl an einen sitzenden als auch an einen liegenden Patienten heranzubringen. Außerdem wird das Nachfüllen von flüssigem Helium erleichtert; dieses wird bei vollständig abgesenktem Meßkryostaten 15 an dessen Oberseite eingefüllt. Aus Gründen der Patienten-und Gerätesicherheit ist an mindestens einer Stativsäule eine Zahnstange angebracht, in welcher ein Rasthebel eingreift, der ein unbeabsichtigtes Absenken des Meßsystems verhindert. Bei Höhenverstellung des Meßsystems muß bewußt die Raste gelöst werden, die dann beim Loslassen durch Federwirkung in die nächste Rastlücke eingreift.

Entlang der Nuten 31 ist der Meßkryostat 15 um den Winkel Alpha verschiebbar. Der maximale Winkel Alpha beträgt dabei ca. 50° nach jeder Seite hin. In den Nuten 31 ist endseitig je ein Anschlag 41 vorgesehen, der ein Anstoßen des geschwenkten Meßkryostaten 15 an die Stativsäule 3 verhindert. Sowohl beim Schwenken um den Winkel Alpha als auch beim Drehen des Bügels bleibt die Applikationsfläche 37 auf den gleichen Punkt P ausgerichtet. Der Punkt P ist in der Regel in den Kopf oder in den Thorax des Patienten gelegt. Mit Hilfe des Schlittens 33 kann der Abstand zwischen der Applikationsfläche 37 und dem Punkt P variiert werden.

Aus der Fig. 2 geht auch noch hervor, daß dem Zahnradantrieb 27 zumindest einseitig ein Schneckengetriebe 43 zugeordnet ist, das ein Schneckenrad 45, eine Schnecke 47 sowie ein Handrad 49 zur Verstellung umfaßt. Dadurch verringert sich der Kraftaufwand für die Winkeleinstellung um den Bolzen 23 und die Einstellung selbst wird genauer. Das gewählte Schneckengetriebe ist ferner selbshemmend. Der Meßkryostat ist also immer fest arretiert. Damit erhöht sich die Patientensicherheit. Dieselben Vorteile ergeben sich mit einem Schneckengetriebe 51 (mit Schneckenrad 53, Schnecke 55 und Handrad 57) zwischen Schlitten 33 und Kreisringsegment 29, mit dessen Hilfe der Schwenkwinkel α verstellt und arretiert werden kann. Die Fig. 2 zeigt, daß der Schlitten 33 in zwei Teile 33a und 33b unterteilt ist. Das Teil 33a sitzt in der Nut 31 und das Teil 33b (z.B. über eine Schwalbenschwanzführung) ist relativ zum Teil 33a in der radialen Richtung verschiebbar. Der Winkel α ergibt sich durch Verschieben des Teiles 33a entlang der Nut 31, wodurch das Teil 33b, das in bestimmter Weise zum Teil 33a radial verschoben ist, mit bewegt wird. Zur Radialverschiebung des Teils 33b relativ zum Teil 33a kann ein weiteres Schneckengetriebe 59 mit dem Schneckenrad 61, der Schnecke 63 und dem Handrad 65 mit den zuvor schon genannten Vorteilen eingesetzt werden.

## Ansprüche

1. Haltevorrichtung für einen Meßkryostaten zur Erfassung biomagnetischer Signale, **dadurch gekennzeichnet,** daß sie zwei Stativsäulen (3) umfaßt, an welchen jeweils ein Laufkasten (17) verschiebbar angeordnet ist, und daß die Laufkästen (17) über einen drehbar gelagerten Bügel (19) miteinander verbunden sind, an welchem der Meßkryostat (15) in der Bügelebene schwenkbar befestigt ist.

2. Haltevorrichtung nach Anspruch 1, **dadurch gekennzeichnet,** daß die beiden Stativsäulen (3) endseitig über eine Querstrebe (5) miteinander verbunden sind.

3. Haltevorrichtung nach Anspruch 2, **dadurch gekennzeichnet,** daß die Querstrebe (5) als Brücke für einen Gewichtsausgleich zwischen dem Meßkryostaten und zwei in den Säulen (3) gleitbar angeordneten Gegengewichten (7) dient.

4. Haltevorrichtung nach einem der Ansprüche 1 bis 3, **dadurch gekennzeichnet,** daß der Bügel (19) an seinen Enden jeweils an einem Kreisscheibensegment (21) befestigt ist, das drehbar um einen an dem zugeordneten Laufkasten (17) befestigten Bolzen (23) gelagert ist.

5. Haltevorrichtung nach Anspruch 4, **dadurch gekennzeichnet,** daß eines der Kreisscheibensegmente (21) an seinem Umfang mit einem Zahnkranz (25) versehen ist, in welchen ein Zahnradantrieb (27) eingreift.

6. Haltevorrichtung nach einem der Ansprüche 1 bis 5, **dadurch gekennzeichnet,** daß in der Mitte des Bügels (19) ein Kreisringsegment (29) mit einer kreissegmentförmigen Nut (31) befestigt ist, an welcher der Meßkryostat (15) in der Bügelebene schwenkbar gelagert ist, wobei das Kreisringsegment (29) parallel zur Bügelebene ausgerichtet ist.

7. Haltevorrichtung nach Anspruch 6, **dadurch gekennzeichnet,** daß der Meßkryostat (15) an dem Kreisringsegment (29) in radialer Richtung verstellbar ist.

8. Haltevorrichtung nach einem der Ansprüche 1 bis 7, **dadurch gekennzeichnet,** daß der Meßkryostat (15) um seine Zentralachse (35) drehbar gelagert ist.

9. Haltevorrichtung nach einem der Ansprüche 6 bis 8, **dadurch gekennzeichnet,** daß das Kreisringsegment (29) an seinen Enden mit je einem Anschlag (41) versehen ist, der ein Anstoßen des geschwenkten Meßkryostaten (15) an der Stativsäule (3) verhindert.

10. Haltevorrichtung nach einem der Ansprüche 4 bis 7, **dadurch gekennzeichnet,** daß zwischen Kreisscheibensegment (21) und Laufkasten (17) ein Schneckengetriebe (43) angeordnet ist.

11. Haltevorrichtung nach Anspruch 6 oder 7, **dadurch gekennzeichnet,** daß zwischen Kreisringsegment (29) und Meßkryostat (15) ein Schneckengetriebe (51) für die Schwenkung ($\alpha$) angeordnet ist.

12. Haltevorrichtung nach Anspruch 7, **dadurch gekennzeichnet,** daß zwischen Kreisringsegment (29) und Meßkryostat (15) ein Schneckengetriebe (59) für die Radialverschiebung angeordnet ist.

86 P 3448

FIG 1

FIG 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB-A- 739 460 (WESTINGHOUSE) <br> * Seite 1, Zeilen 57-79; Figur 1 * <br> ----- | | G 01 R 33/00 <br> A 61 B 5/04 |
| | | | **RECHERCHIERTE SACHGEBIETE (Int. Cl.4)** <br><br> G 01 R <br> G 12 B <br> A 61 B |

Der vorliegende Recherchenbericht wurde für alle Patentansprüche erstellt

| Recherchenort | Abschlußdatum der Recherche | Prüfer |
|---|---|---|
| DEN HAAG | 21-03-1988 | HAASBROEK J.N. |